# EUROPEAN PATENT SPECIFICATION

(11) **EP 4 262 710 B1**
(45) Date of publication and mention of the grant of the patent: **22.05.2024**
(21) Application number: 22707752.6
(22) Date of filing: 25.02.2022
(51) Int. Cl.: A61K 8/34, A61K 8/60, A61Q 5/08, A61Q 5/10, A61K 8/23

(54) **BLEACHING COMPOSITION FOR KERATIN FIBERS**
BLEICHZUSAMMENSETZUNG FÜR KERATINFASERN
COMPOSITION DE BLANCHIMENT POUR FIBRES DE KÉRATINE

(30) Priority: 26.02.2021 EP 21159448
(43) Date of publication of application: 25.10.2023
(73) Proprietor: Kao Germany GmbH, 64297 Darmstadt (DE)
(72) Inventor: BREAKSPEAR, Steven, 64297 Darmstadt (DE); BAUER, Peter, 64297 Darmstadt (DE); NÖCKER, Bernd, 64297 Darmstadt (DE); RABELO DE MORAES, Ines, 64297 Darmstadt (DE)
(74) Representative: Miller, Tobias
(86) International application number: PCT/EP2022/054764
(87) International publication number: WO 2022/180202

(56) References cited:
- EP-A1- 3 040 065
- US-A- 5 294 436
- DATABASE GNPD [Online] MINTEL; 25 September 2020 (2020-09-25), anonymous: "Bleaching Powder", XP055836499, Database accession no. 8134959
- DATABASE GNPD [Online] MINTEL; 23 September 2020 (2020-09-23), anonymous: "Bleach, Please Complete Hair Lightening Kit", XP055836500, Database accession no. 8125919
- DATABASE GNPD [Online] MINTEL; 20 August 2004 (2004-08-20), anonymous: "Ex Hi-Blech Hair Colourant", XP055836501, Database accession no. 295446

## Description

### Field of the invention

The invention is directed to the provision of a bleaching composition for keratin fibers, a kit-of-parts for bleaching, and a method for bleaching keratin fibers.

### Background of the invention

Many consumers are unsatisfied with their hair color. Changes of hair color, in most cases, require the lightening of hair, especially for dark hair customers. A common technique is hair bleaching of whole hair as well partial treatments such as streaks. For such treatments a high degree of lightening is desired.

One way to increase lightening performance of bleaching compositions is the use of a reductive pre-treatment step, e.g. by using organic thiols (EP 2 608 848 B1) or inorganic reducing agents such as sulfites. However, with a reductive pre-treatment step hair lightening and hair damage are increased simultaneously.

EP0525239 discloses the use of acetylated sugar alcohols at concentrations of 45 by weight or more to increase the performance of perborate bleach for textile fibers.

EP3040065 discloses the use of mono- and disaccharides in aqueous oxidizing composition.

US5294436 A discloses a highlighting method for hair using application of a powder bleach followed by misting with hydrogen peroxide solution.

Thus, there is a real need to develop bleaching composition, which deliver an increased degree of lightening without leading to high degrees of damage.

### Summary of the invention

Therefore, the first object of the present invention is a bleaching composition A for keratin fibers, preferably for human keratin fibers, more preferably for human hair, com prising
a) one or more persalt(s) and/or peroxy salt(s),
b) one or more alkalizing agent(s),
c) one or more non-acetylated sugar alcohol(s), and/or their mixtures,

wherein the total concentration of compound(s) according to group c) is in the range of 0.0001 % to 2.5% by weight, calculated to the total weight of the bleaching com position A,
wherein the bleaching composition A com prises less than 5% by weight of water, calculated to the total weight of the bleaching composition A,
and wherein one or more compound(s) according to group c) is/are mannitol, xylitol, and/or their m ixtures.

The second object of the present invention is a two-part bleaching composition for keratin fibers, preferably for human keratin fibers, more preferably for human hair, comprising a bleaching composition A as defined above and a second aqueous oxidizing composition B, preferably comprising hydrogen peroxide and having a pH in the range of 1 to 6.

The third object of the present invention is a kit-of-parts for bleaching and dyeing keratin fibers, preferably human keratin fibers, more preferably human hair com prising:
- a bleaching composition A as defined above,
- a second aqueous oxidizing composition B, preferably comprising hydrogen peroxide and having a pH in the range of 1 to 6,
- a third composition comprising one or more dye(s), preferably one or more oxidative hair dye(s) and/or one or more hair direct dye(s), and/or their mixtures.

The fourth object of the present invention is a method for bleaching keratin fibers, preferably human keratin fibers, more preferably human hair, comprising the steps of:
i) mixing a bleaching composition A as defined above with a second oxidizing composition B, preferably comprising hydrogen peroxide and having a pH in the range of 1 to 6, to yield a ready-to-use composition having a pH in the range of 7 to 12,
ii) applying the ready-to-use composition onto keratin fibers and leaving it for a time period of 1 min to 60 min,
iii) rinsing-off the keratin fibers with water and optionally shampooing the keratin fibers.

A fifth object of the present invention is a method for dyeing of keratin fibers, preferably human keratin fibers, more preferably human hair, comprising the steps of:
iv) providing the bleaching composition A as defined above and mixing it with an aqueous oxidizing composition B, preferably comprising hydrogen peroxide and having a pH in the range of 1 to 6, to yield a ready-to-use composition having a pH in the range of 7 to 12,
v) applying the ready-to-use composition onto keratin fibers and leaving it for a time period of 1 min to 60 min,
vi) rinsing-off the keratin fibers with water and optionally shampooing the keratin fibers,
vii) providing a dyeing composition comprising one or more dye(s) selected from oxidative dye coupler, oxidative dye precursor, and direct dye, and/or their mixtures, and comprising one or more alkalizing agent(s),
viii) mixing the composition of step vii) with an aqueous oxidative composition B to form a ready-to-use dyeing composition having a pH in the range of 7 to 12,
ix) applying the ready-to-use dyeing composition onto keratin fibers and leaving it for a time period of 1 min to 60 min,
x) repeating step vi).

### Detailed description of the invention

Inventors of the present invention have unexpectedly found out that the objects of the present invention increase bleaching performance while not leading to elevated damage of keratin fibers. Moreover, due to the mildness of the compositions to fibers, they feel more cosmetic, healthier, and appear to have more shine.

### Bleaching composition A

The present invention is directed to a bleaching composition A for keratin fibers, preferably for human keratin fibers, more preferably for human hair, comprising
a) one or more persalt(s) and/or peroxy salt(s),
b) one or more alkalizing agent(s),
c) one or more non-acetylated sugar alcohol(s), and/or their mixtures,

wherein the total concentration of compound(s) according to group c) is in the range of 0.0001 % to 2.5% by weight, calculated to the total weight of the bleaching com position A,
wherein the bleaching composition A com prises less than 5% by weight of water, calculated to the total weight of the bleaching composition A,
and wherein one or more compound(s) according to group c) is/are mannitol, xylitol, and/or their m ixtures.

Preferably, the bleaching composition A comprises less than 1% by weight of water, more preferably it is an anhydrous composition, from the viewpoint of stability. The term anhydrous is to be understood that no water is added to the powder. However, this does not exclude any water bound to the ingredients by, for example, capillary forces.

### Compound(s) according to group a)

The bleaching composition A comprises one or more persalt(s) and/or peroxy salt(s) as compound(s) according to group a). Suitable persalts and/or peroxy salts are sodium persulfate, potassium persulfate, ammonium persulfate, earth alkali peroxides such as magnesium peroxide, melamine peroxide or urea peroxide or phthalimidoperoxy hexanoic acid. The preferred persalts from the viewpoint of bleaching power are sodium, potassium, and ammonium persulfate.

It is preferred from the viewpoint of bleaching power and cosmetic safety that the total concentration of persalts and/or peroxy salts in the bleaching composition A is in the range of 10% to 80% by weight, preferably 15% to 70% by weight, more preferably 20% to 60% by weight, and still more preferably 25% to 60% by weight, calculated to the total weight of the bleaching composition A.

### Compound(s) according to group b)

The bleaching composition A further comprises one or more alkalizing agent(s) as compounds according to group b). Preferably, the compounds according to group b) are selected from inorganic and/or organic alkalizing agent(s), and/or their mixtures.

It is preferred from the viewpoint of stability and bleaching power that the compounds according to group b) are inorganic alkalizing agent(s), preferably selected from metasilicates, carbonates, and/or bicarbonates, and/or their alkali or earth alkali salts, and/or their mixtures, more preferably the compound according to group b) is sodium metasilicate.

It is preferred from the viewpoint of alkalinity that the compounds according to group b) are organic alkalizing agent(s), preferably selected from alkyl- or alkanolamines according to the general structure 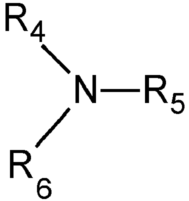 wherein R₄, R₅, and R₆ are same or different H, from C₁ to C₄, C₃ to C₄ unsaturated alkyl, C₃ to C₄ branched alkyl, C₁ to C₄ hydroxyl alkyl, C₃ to C₄ unsaturated hydroxyl alkyl, C₃ to C₄ branched hydroxyl alkyl, with the condition that at least one of R₄, R₅, or R₆ is different from H, and/or their mixtures.

Suitable organic alkalizing agents are monoethanolamine, diethanolamine, triethanolamine, monomethylamine, dimethylamine, trimethylamine, monoethylamine, diethylamine, and 2-aminomethyl propanol.

The most preferred organic alkalizing agent(s) as compounds according to group b) are selected from monoethanolamine and/or 2-aminomethyl propanol.

It is preferred from the viewpoint of alkalinity and stability that the composition comprises one or more compound according to group b) at a total concentration in the range of 0.25% to 30% by weight, preferably 0.5% to 25% by weight, more preferably 1 % to 20% by weight, calculated to the total weight of the bleaching com position A.

Optionally, the bleaching composition A may comprise one or more ammonium salt(s) different from persalt(s) and peroxy salt(s).

Suitable ammonium salts different from persalt(s) and peroxy salt(s) are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. The bleaching composition A may also comprise mixtures of ammonium salts.

The bleaching composition A may comprise one or more ammonium salts different from persalt(s) and peroxy salt(s) at a total concentration in the range of 0.1% to 10% by weight, calculated to the total weight of the composition.

### Compound(s) according to group c)

The bleaching composition A of the present invention comprises one or more non-acetylated sugar alcohol(s), and/or their mixtures, as compound(s) according to group c), wherein the total concentration of compound(s) according to group c) is in the range of 0.0001% to 2.5% by weight, calculated to the total weight of the bleaching composition A.

It is further preferred from the viewpoint of bleaching power that the one or more non-acetylated sugar alcohol(s) as compound(s) according to group c) is/are mannitol, xylitol, and/or their mixtures, preferably it is mannitol.

It is further preferred from the viewpoint of bleaching power that the total concentration of compound(s) according to group c) is in the range of 0.0005% to 0.5% by weight, preferably in the range of 0.001% to 0.25% by weight, more preferably in the range of 0.001% to 0.1% by weight, calculated to the total weight of the bleaching composition A.

The disclosure of the present invention also includes a bleaching composition A for keratin fibers, preferably for human keratin fibers, more preferably for human hair, com prising
a) one or more persalt(s) and/or peroxy salt(s),
b) one or more alkalizing agent(s),
c) one or more non-acetylated sugar alcohol(s), and/or their mixtures,

wherein the total concentration of compound(s) according to group c) is in the range of 0.0001% to 2.5% by weight, preferably in the range of 0.0001% to 1% by weight, more preferably in the range of 0.0001% to 0.1% by weight, calculated to the total weight of the bleaching composition A, and
wherein the bleaching composition A com prises less than 1 % by weight of water, calculated to the total weight of the bleaching composition A, preferably it is anhydrous.

### Cosmetic forms of bleaching composition A

The bleaching composition A of the present invention may be in the form of a bleaching powder composition.

For preparation of the bleaching powder composition, an excipient may be added. Such an excipient is diatomaceous earth.

It is further preferred from the viewpoint of cosmetic safety that the bleaching powder composition is dust-free. This property can commonly be achieved by adding lipophilic compounds to the bleaching powder. From this viewpoint, the composition comprises one or more lipophilic compound(s) as compound according to group d).

The bleaching composition A may be in the form of a bleaching paste composition. It is further preferred that the bleaching paste comprises one or more lipophilic compound(s) as compound according to group d).

Preferably, the compound according to group d) is selected from C₁₂ to C₂₂ fatty alcohols, esters of C₃ to C₁₂ alcohols with C₁₂ to C₂₂ fatty acids, C₈ to C₂₂ fatty acids, vegetable oils, and/or silicones, and/or hydrocarbon-based products, and/or their mixtures.

Suitable C₁₂ to C₂₂ fatty alcohols are are myristyl alcohol, cetyl alcohol, stearyl alcohol, behenyl alcohol, and cetearyl alcohol.

Suitable esters of C₃ to C₂₂ alcohols with C₁₂ to C₂₂ fatty acids are isopropyl myristate, isopropyl palitate, and myristyl myristate.

Suitable C₈ to C₂₂ fatty acids are oleic acid, linoleic acid, and palm itic acid.

Suitable vegetable oils are olive oil, almond oil, sunflower oil, and argan oil.

Suitable silicones are non-aminated and/or aminated silicones. The latter are commonly known as amodimethicones.

Suitable hydrocarbon-based products are mineral oil, paraffins, and Vaseline.

It is preferred from the viewpoint of making the bleaching composition A dust-free or formulating it as a paste that the concentration of compounds according to group d) is in the range of 1% to 20% by weight, preferably 2% to 15% by weight, more preferably 3% to 12% by weight, calculated to the total weight of the bleaching com position A.

### Optional compounds - surfactants as compounds according to group e)

The bleaching composition A of the present invention may further comprise one or more surfactant(s) as compound according to group e), preferably selected from non-ionic surfactants, anionic surfactants, cationic surfactants, and/or amphoteric/zwitterionic surfactants, and/or their mixtures, more preferably selected from anionic surfactants and/or non-ionic surfactants, from the viewpoint of stabilizing the composition and improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures.

Suitable examples are alkyl sulfate or preferably ethoxylated alkyl ether sulfate surfactant or mixtures thereof having an alkyl chain length of C₁₀ to C₂₂.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants. Suitable examples are cetrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of the bleaching composition A.

### Optional compounds - thickening polymers

In case the viscosity after mixing with other compositions needs to be further adjusted to prevent dripping, the bleach powder composition may comprise one or more thickening polymers, from the viewpoint of cosmetic safety.

The composition of the present invention comprises one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH between 7 and 12 having a viscosity of at least 1,000 mPa•s measured at a polymer concentration of 1% by weight in water at 25°C, calculated to the total weight of the composition, determined by a Brookfield viscometer, such as at 10 rpm for 1 min, with an appropriate spindle at 25°C.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers in the bleaching composition A is 0.1% by weight or more, more preferably 0.25% by weight or more, more preferably 0.5% by weight or more, calculated to the total weight of the bleaching composition A, from the viewpoint of providing sufficient viscosity to the com position.

Preferably, the total concentration of thickening polymers of the bleaching composition A is 15% by weight or less, more preferably 12% by weight or more, further more preferably 10% by weight or less, calculated to the total weight of the composition, from the viewpoint of providing sufficient viscosity to the composition and cost of goods.

For attaining the above-mentioned effects, it is preferred that the total concentration of thickening polymers in the bleaching composition A of the present invention is in the range of 0.1% to 15% by weight, preferably 0.25% to 12% by weight, more preferably in the range of 0.5% to 10% by weight, calculated to the total weight of the bleaching composition A.

### Two-part composition

The present invention is also directed to a two-part bleaching composition for keratin fibers, preferably for human keratin fibers, more preferably for human hair, comprising a bleaching composition A as defined above and a second aqueous oxidizing composition B, preferably comprising hydrogen peroxide and having a pH in the range of 1 to 6.

Suitable concentrations of the oxidizing agent(s), preferably hydrogen peroxide, in the aqueous oxidizing composition B is in the range of 1% to 20% by weight, more preferably 2% to 15% by weight, further more preferably 3% to 12% by weight, calculated to the total weight of the second oxidizing composition B.

Suitably, from the viewpoint of stability of the aqueous oxidizing composition B, the pH of the composition is in the range of 1 to 6, more preferably 1.5 to 5, more preferably 2 to 4.5.

The pH may be adjusted with well-known acids such as phosphoric acid.

### Kit-of-parts

The present invention is also directed to a kit-of-parts for bleaching and dyeing keratin fibers, preferably human keratin fibers, more preferably human hair com prising:
- a bleaching composition A as defined above,
- a second aqueous oxidizing composition B, preferably comprising hydrogen peroxide and having a pH in the range of 1 to 6,
- a third composition comprising one or more dye(s), preferably one or more oxidative hair dye(s) and/or one or more hair direct dye(s), and/or their mixtures.

The second aqueous oxidizing composition may have the same features and characteristics as the second aqueous composition B of the two-part composition.

The third composition comprising one or more dye(s), preferably one or more oxidative hair dye(s) and/or one or more hair direct dye(s), and/or their mixtures may further comprise one or more alkalizing agent(s). Preferably, this composition has a pH in the range of 7 to 12, more preferably in the range of 8 to 11, further more preferably 8.5 to 10.

Suitable alkalizing agents are ammonia and the alkalizing agents as present above for the bleaching composition A. The concentration of alkalizing agents in the dyeing composition may be similar to the bleaching composition A.

### Method for bleaching

The present invention is also directed to a method for bleaching keratin fibers, preferably human keratin fibers, more preferably human hair, comprising the steps of:
i) mixing a bleaching composition A as defined above with a second oxidizing composition B, preferably comprising hydrogen peroxide and having a pH in the range of 1 to 6, to yield a ready-to-use composition having a pH in the range of 7 to 12,
ii) applying the ready-to-use composition onto keratin fibers and leaving it for a time period of 1 min to 60 min,
iii) rinsing-off the keratin fibers with water and optionally shampooing the keratin fibers.

The bleaching composition A is mixed with the aqueous oxidizing composition B to form a ready-to-use composition. Suitable mixing ratios by weight are 5:1 to 1:5 (bleach powder composition: aqueous oxidizing composition). Customarily, suitable mixing ratios are 1:1, 1:2, and 1:3 by weight (bleaching composition A: aqueous oxidizing composition B).

Suitably, the pH of the ready-to-use composition is in the range of 7 to 12. It is preferred from the viewpoint of accelerated bleaching that the pH of the ready-to-use composition is in the range of 7.5 to 11, more preferably 8.0 to 10.5.

The ready-to-use composition is then applied to keratin fibers and left for a time period of 1 min to 60 min as defined in step ii). Preferred time ranges for step ii) are 5 min to 50 min, more preferred ranges are 10 min to 45 min, from the viewpoint of sufficiently bleaching the keratin fibers.

After that, the ready-to-use composition is rinsed-off from keratin fibers and optionally they are shampooed and optionally blow-dried.

### Method for dyeing

The present invention is also directed to a method for dyeing of keratin fibers, preferably human keratin fibers, more preferably human hair, comprising the steps of:
iv) providing the bleaching composition A as defined above and mixing it with an aqueous oxidizing composition B, preferably comprising hydrogen peroxide and having a pH in the range of 1 to 6, to yield a ready-to-use composition having a pH in the range of 7 to 12,
v) applying the ready-to-use composition onto keratin fibers and leaving it for a time period of 1 min to 60 min,
vi) rinsing-off the keratin fibers with water and optionally shampooing the keratin fibers,
vii) providing a dyeing composition comprising one or more oxidative dye(s) and/or direct dye(s), and/or their mixtures, and comprising one or more alkalizing agent(s),
viii) mixing the composition of step vii) with an aqueous oxidative composition B to form a ready-to-use dyeing composition having a pH in the range of 7 to 12,
ix) applying the ready-to-use dyeing composition onto keratin fibers and leaving it for a time period of 1 min to 60 min,
x) repeating step vi).

Process steps iv) to vi) are identical to process steps i) to iii) and, therefore, the disclosure of the bleaching process is essentially the same for the bleaching process in combination with the oxidative dyeing process.

The oxidative dyeing composition of step vii) may be the same composition as disclosed for the kit-of-parts.

The following examples are to illustrate the invention, but not to limit it.

### EXAMPLES

**Table 1**

| **Bleaching composition A** | | |
|---|---|---|
| **Ingredient** | **Inventive example 1** | **Comparative example 1** |
| | **% by weight** | |
| **Potassium persulfate** | 40.0 | 40.0 |
| **Ammonium persulfate** | 15.0 | 15.0 |
| **Sodium metasilicate** | 11.0 | 11.0 |
| **Mannitol** | 0.1 | - |
| **Diatomaceous earth** | Ad 100.0 | |
| | | |
| **L*** | 40.73 | 36.73 |

### Methods

### Bleaching method

For table 1:
Caucasian hair streaks (21 cm, 2 g per bundle) were purchased from Fischbach + Miller Haar, Laupheim, Germany. The bleaching compositions A of table 1 from above were mixed in a weight ratio of 1:1.4 (bleaching composition A: aqueous oxidizing composition B) with an aqueous oxidizing composition B comprising 6% by weight of hydrogen peroxide to prepare a ready-to-use composition with a pH of 10.0 ± 0.2. 1 g of the ready-to-use compositions were applied onto hair streaks and left for 30 min at room temperature. The hair streaks were then rinsed-off with lukewarm water, shampooed with a shampoo commercially available under the trade name Goldwell Deep Cleansing Shampoo, and blow-dried.

### Lightening measurements

L* values were measured after bleaching with a Datacolor 45G instrument.

The following examples are within the scope of the present invention.

### Example 2

### Bleaching composition A

| | **% by weight** |
|---|---|
| Hydroxyethylcellulose | 3 |
| Tetrasodium EDTA | 2 |
| Sodium carbonate | 1 |
| Ammonium persulfate | 11 |
| Potassium persulfate | 36 |
| Sodium metasilicate | 10 |
| Mannitol | 0.001 |
| Mineral oil | 8 |
| Diatomaceous Earth | to 100 |

### Example 3

### Two-part composition

### Bleaching composition A

| | **% by weight** |
|---|---|
| Hydroxyethylcellulose | 3 |
| Tetrasodium EDTA | 2 |
| Sodium carbonate | 1 |
| Ammonium persulfate | 11 |
| Potassium persulfate | 36 |
| Sodium metasilicate | 10 |
| Mannitol | 0.5 |
| Mineral oil | 8 |
| Diatomaceous Earth | to 100 |

### Aqueous oxidative composition B

| | **% by weight** |
|---|---|
| Cetearyl alcohol | 4.0 |
| Sodium lauryl sulfate | 0.8 |
| Phosphoric acid | q.s. ad pH 2.5 |
| Tetrasodium EDTA | 0.05 |
| Hydrogen peroxide | 6.0 |
| Water | ad 100.0 |

### Example 4

### Kit for bleaching/lightening and oxidative dyeing

The bleaching composition A and aqueous oxidative composition B of example 3 are combined with the following oxidative dyeing composition:

| | **% by weight** |
|---|---|
| Cetearyl alcohol | 12 |
| Sodium cetearyl sulfate | 2 |
| Cocamide MEA | 5 |
| Oleic acid | 2 |
| Tetrasodium EDTA | 1 |
| Sodium sulfite | 1 |
| Ammonium hydroxide | 5 |
| Ammonium chloride | 1 |
| Toluene-2,5-Diamine sulfate | 0.75 |
| Resorcinol | 0.10 |
| 4-Chlorresorcinol | 0.25 |
| m-Aminophenol | 0.05 |
| 4-Amino-2-Hydroxytoluene | 0.05 |
| Fragrance | 0.5 |
| Water | ad 100 |

The above composition is adjusted to 9.5 by addition of NaOH/HCl.

## Claims

1. A bleaching composition A for keratin fibers, preferably for human keratin fibers, more preferably for human hair, comprising
a) one or more persalt(s) and/or peroxy salt(s),
b) one or more alkalizing agent(s),
c) one or more non-acetylated sugar alcohol(s), and/or their mixtures,
wherein the total concentration of compound(s) according to group c) is in the range of 0.0001% to 2.5% by weight, calculated to the total weight of the bleaching composition A,
wherein the bleaching composition A comprises less than 5% by weight of water, calculated to the total weight of the bleaching composition A,
and wherein one or more compound(s) according to group c) is/are mannitol, xylitol, and/or their mixtures.

2. The composition according to claim 1 **characterized in that** it comprises compound(s) according to group a) at a total concentration in the range of 10% to 80% by weight, preferably 15% to 70% by weight, more preferably 20% to 60% by weight, still more preferably 25% to 60% by weight, calculated to the total weight of composition A.

3. The composition according to claims 1 and/or 2 **characterized in that** the compounds according to group b) are inorganic alkalizing agent(s), preferably selected from metasilicates, carbonates, and/or bicarbonates, and/or their alkali or earth alkali salts, and/or their mixtures, more preferably the compound according to b) is sodium metasilicate.

4. The composition according to any of the preceding claims **characterized in that** the compounds according to group b) are organic alkalizing agent(s), preferably selected from, alkyl- or alkanolamines according to the general structure 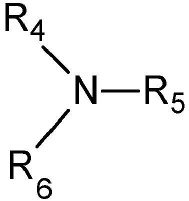 wherein R₄, R₅, and R₆ are same or different H, from C₁ to C₄, C₃ to C₄ unsaturated alkyl, C₃ to C₄ branched alkyl, C₁ to C₄ hydroxyl alkyl, C₃ to C₄ unsaturated hydroxyl alkyl, C₃ to C₄ branched hydroxyl alkyl, with the condition that at least one of R₄, R₅, or R₆ is different from H, and/or their mixtures, more preferably the compounds according to b) are selected from monoethanolamine and/or 2-aminomethyl propanol.

5. The composition according any of the preceding claims **characterized in that** the composition comprises one or more compound according to group b) at a total concentration in the range of 0.25% to 30% by weight, preferably 0.5% to 25% by weight, more preferably 1% to 20% by weight, calculated to the total weight of composition A.

6. The composition according to any of the preceding claims **characterized in that** the one or more non-acetylated sugar alcohol(s) as compound(s) according to group c) is mannitol.

7. The composition according to any of the preceding claims **characterized in that** the total concentration of compound(s) according to group c) is in the range of 0.0005% to 0.5% by weight, preferably in the range of 0.001% to 0.25% by weight, more preferably in the range of 0.001% to 0.1% by weight, calculated to the total weight of composition A.

8. The composition according to any of the preceding claims **characterized in that** it is a bleaching powder composition or
a bleaching paste composition.

9. The composition according to any of the preceding claims **characterized in that** it comprises one or more lipophilic compound(s) as compound according to group d).

10. The composition according to claim 9 **characterized in that** the compound according to group d) is selected from C₁₂ to C₂₂ fatty alcohols, esters of C₃ to C₁₂ alcohols with C₁₂ to C₂₂ fatty acids, C₈ to C₂₂ fatty acids, vegetable oils, and/or silicones, and/or hydrocarbon-based products, and/or their mixtures.

11. The composition according to any of the claims 9 to 10 **characterized in that** the concentration of compounds according to group d) is in the range of 1% to 20% by weight, preferably 2% to 15% by weight, more preferably 3% to 12% by weight, calculated to the total weight of composition A.

12. A two-part bleaching composition for keratin fibers, preferably for human keratin fibers, more preferably for human hair, comprising a bleaching composition A according to any of the claims 1 to 11 and a second aqueous oxidizing composition B, preferably comprising hydrogen peroxide and having a pH in the range of 1 to 6.

13. A kit-of-parts for bleaching and dyeing keratin fibers, preferably human keratin fibers, more preferably human hair comprising:
- a bleaching composition A as defined in any of the claims 1 to 11,
- a second aqueous oxidizing composition B, preferably comprising hydrogen peroxide and having a pH in the range of 1 to 6,
- a third composition comprising one or more dye(s), preferably one or more oxidative hair dye(s) and/or one or more hair direct dye(s), and/or their mixtures.

14. A method for bleaching keratin fibers, preferably human keratin fibers, more preferably human hair, comprising the steps of:
i) mixing a bleaching composition A as defined in any of the claims 1 to 11 with a second oxidizing composition B, preferably comprising hydrogen peroxide and having a pH in the range of 1 to 6, to yield a ready-to-use composition having a pH in the range of 7 to 12,
ii) applying the ready-to-use composition onto keratin fibers and leaving it for a time period of 1 min to 60 min,
iii) rinsing-off the keratin fibers with water and optionally shampooing the keratin fibers.

15. A method for dyeing of keratin fibers, preferably human keratin fibers, more preferably human hair, comprising the steps of:
iv) providing the bleaching composition A as defined in any of the claims 1 to 11 and mixing it with an aqueous oxidizing composition B, preferably comprising hydrogen peroxide and having a pH in the range of 1 to 6, to yield a ready-to-use composition having a pH in the range of 7 to 12,
v) applying the ready-to-use composition onto keratin fibers and leaving it for a time period of 1 min to 60 min,
vi) rinsing-off the keratin fibers with water and optionally shampooing the keratin fibers,
vii) providing a dyeing composition comprising one or more oxidative dye(s) and/or direct dye(s), and/or their mixtures, and comprising one or more alkalizing agent(s),
viii) mixing the composition of step vii) with an aqueous oxidative composition B to form a ready-to-use dyeing composition having a pH in the range of 7 to 12,
ix) applying the ready-to-use dyeing composition onto keratin fibers and leaving it for a time period of 1 min to 60 min,
x) repeating step vi).

## Patentansprüche

1. Bleichmittelzusammensetzung A für Keratinfasern, vorzugsweise für menschliche Keratinfasern, besonders bevorzugt für menschliches Haar, umfassend
a) ein oder mehrere Persalz(e) und/oder Peroxysalz(e),
b) einem oder mehreren Alkalisierungsmittel(n),
c) einen oder mehrere nicht-acetylierte Zuckeralkohole und/oder deren Mischungen,
wobei die Gesamtkonzentration der Verbindung(en) gemäß Gruppe c) im Bereich von 0.0001 Gew.-% bis 2.5 Gew.-%, berechnet auf das Gesamtgewicht der Bleichmittelzusammensetzung A, liegt,
wobei die Bleichmittelzusammensetzung A weniger als 5 Gew.-% Wasser, bezogen auf das Gesamtgewicht der Bleichmittelzusammensetzung A, enthält,
und wobei eine oder mehrere Verbindung(en) gemäß Gruppe c) Mannit, Xylit und/oder deren Mischungen ist/sind.

2. Zusammensetzung nach Anspruch 1, **dadurch gekennzeichnet, dass** sie Verbindung(en) gemäß Gruppe a) in einer Gesamtkonzentration im Bereich von 10 bis 80 Gew.-%, vorzugsweise 15 bis 70 Gew.-%, noch bevorzugter 20 bis 60 Gew.-%, noch bevorzugter 25 bis 60 Gew.-%, berechnet auf das Gesamtgewicht der Zusammensetzung A, enthält.

3. Zusammensetzung nach Anspruch 1 und/oder 2, **dadurch gekennzeichnet, dass** es sich bei den Verbindungen nach Gruppe b) um anorganische Alkalisierungsmittel handelt, die vorzugsweise aus Metasilikaten, Carbonaten und/oder Bicarbonaten und/oder deren Alkali- oder Erdalkalisalzen und/oder deren Gemischen ausgewählt sind, wobei die Verbindung nach b) vorzugsweise Natriummetasilikat ist.

4. Zusammensetzung nach einem der vorhergehenden Ansprüche, **dadurch gekennzeichnet, dass** die Verbindungen nach Gruppe b) organische Alkalisierungsmittel sind, vorzugsweise ausgewählt aus Alkyl- oder Alkanolaminen der allgemeinen Struktur 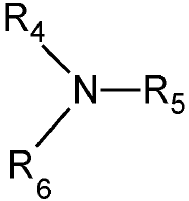 worin R₄, R₅ und R₆ gleich oder verschieden sind von H, von C₁ bis C₄, C₃ bis C₄ ungesättigtem Alkyl, C₃ bis C₄ verzweigtem Alkyl, C₁ bis C₄ Hydroxylalkyl, C₃ bis C₄ ungesättigtem Hydroxylalkyl, C₃ bis C₄ verzweigtem Hydroxylalkyl, mit der Bedingung, dass mindestens einer der Reste R₄, R₅ oder R₆ von H verschieden ist, und/oder deren Mischungen, besonders bevorzugt sind die Verbindungen gemäß b) ausgewählt aus Monoethanolamin und/oder 2-Aminomethylpropanol.

5. Zusammensetzung nach einem der vorhergehenden Ansprüche, **dadurch gekennzeichnet, dass** die Zusammensetzung eine oder mehrere Verbindungen gemäß Gruppe b) in einer Gesamtkonzentration im Bereich von 0.25 bis 30 Gew.-%, vorzugsweise 0.5 bis 25 Gew.-%, noch bevorzugter 1 bis 20 Gew.-%, bezogen auf das Gesamtgewicht der Zusammensetzung A, enthält.

6. Zusammensetzung nach einem der vorhergehenden Ansprüche, **dadurch gekennzeichnet, dass** der eine oder mehrere nicht-acetylierte Zuckeralkohol(e) als Verbindung(en) nach Gruppe c) Mannitol ist.

7. Zusammensetzung nach einem der vorhergehenden Ansprüche, **dadurch gekennzeichnet, dass** die Gesamtkonzentration der Verbindung(en) gemäß Gruppe c) im Bereich von 0.0005 Gew.-% bis 0.5 Gew.-%, vorzugsweise im Bereich von 0.001 Gew.-% bis 0.25 Gew.-%, besonders bevorzugt im Bereich von 0.001 Gew.-% bis 0.1 Gew.-%, bezogen auf das Gesamtgewicht der Zusammensetzung A, liegt.

8. Zusammensetzung nach einem der vorhergehenden Ansprüche, **dadurch gekennzeichnet, dass** sie eine Bleichpulverzusammensetzung ist oder eine Bleichpastenzusammensetzung.

9. Zusammensetzung nach einem der vorhergehenden Ansprüche, **dadurch gekennzeichnet, dass** sie eine oder mehrere lipophile Verbindung(en) als Verbindung nach Gruppe d) enthält.

10. Zusammensetzung nach Anspruch 9, **dadurch gekennzeichnet, dass** die Verbindung gemäß Gruppe d) ausgewählt ist aus C₁₂ bis C₂₂ Fettalkoholen, Estern von C₃ bis C₁₂ Alkoholen mit C₁₂ bis C₂₂ Fettsäuren, C₈ bis C₂₂ Fettsäuren, pflanzlichen Ölen und/oder Silikonen und/oder Produkten auf Kohlenwasserstoffbasis und/oder deren Mischungen.

11. Zusammensetzung nach einem der Ansprüche 9 bis 10, **dadurch gekennzeichnet, dass** die Konzentration der Verbindungen gemäß Gruppe d) im Bereich von 1 bis 20 Gew.-%, vorzugsweise 2 bis 15 Gew.-%, besonders bevorzugt 3 bis 12 Gew.-%, bezogen auf das Gesamtgewicht der Zusammensetzung A, liegt.

12. Zweiteilige Bleichzusammensetzung für Keratinfasern, vorzugsweise für menschliche Keratinfasern, noch bevorzugter für menschliches Haar, umfassend eine Bleichzusammensetzung A nach einem der Ansprüche 1 bis 11 und eine zweite wässrige oxidierende Zusammensetzung B, die vorzugsweise Wasserstoffperoxid enthält und einen pH-Wert im Bereich von 1 bis 6 aufweist.

13. Ein Kit zum Bleichen und Färben von Keratinfasern, vorzugsweise menschlichen Keratinfasern, besonders bevorzugt menschlichem Haar, umfassend:
- eine Bleichmittelzusammensetzung A nach einem der Ansprüche 1 bis 11,
- eine zweite wässrige oxidierende Zusammensetzung B, die vorzugsweise Wasserstoffperoxid enthält und einen pH-Wert im Bereich von 1 bis 6 aufweist,
- eine dritte Zusammensetzung, die einen oder mehrere Farbstoffe, vorzugsweise einen oder mehrere oxidative Haarfärbemittel und/oder einen oder mehrere Haardirektfarbstoffe und/oder deren Mischungen enthält.

14. Verfahren zum Bleichen von Keratinfasern, vorzugsweise menschlichen Keratinfasern, besonders bevorzugt menschlichem Haar, das die folgenden Schritte umfasst:
i) Mischen einer Bleichzusammensetzung A, wie in einem der Ansprüche 1 bis 11 definiert, mit einer zweiten oxidierenden Zusammensetzung B, die vorzugsweise Wasserstoffperoxid umfasst und einen pH-Wert im Bereich von 1 bis 6 aufweist, um eine gebrauchsfertige Zusammensetzung mit einem pH-Wert im Bereich von 7 bis 12 zu erhalten,
ii) Auftragen der gebrauchsfertigen Zusammensetzung auf die Keratinfasern und Einwirkenlassen für eine Zeitspanne von 1 min bis 60 min,
iii) Abspülen der Keratinfasern mit Wasser und gegebenenfalls Shampoonieren der Keratinfasern.

15. Verfahren zum Färben von Keratinfasern, vorzugsweise menschlichen Keratinfasern, besonders bevorzugt menschlichem Haar, das die folgenden Schritte umfasst:
iv) Bereitstellen der Bleichmittelzusammensetzung A, wie in einem der Ansprüche 1 bis 11 definiert, und Mischen mit einer wässrigen oxidierenden Zusammensetzung B, die vorzugsweise Wasserstoffperoxid enthält und einen pH-Wert im Bereich von 1 bis 6 aufweist, um eine gebrauchsfertige Zusammensetzung mit einem pH-Wert im Bereich von 7 bis 12 zu erhalten,
v) Auftragen der gebrauchsfertigen Zusammensetzung auf die Keratinfasern und Einwirkenlassen für eine Zeitspanne von 1 min bis 60 min,
vi) Abspülen der Keratinfasern mit Wasser und gegebenenfalls Shampoonieren der Keratinfasern,
vii) Bereitstellen einer Färbezusammensetzung, die einen oder mehrere oxidative(n) Farbstoff(e) und/oder Direktfarbstoff(e) und/oder deren Mischungen und ein oder mehrere Alkalisierungsmittel enthält,
viii) Mischen der Zusammensetzung von Schritt vii) mit einer wässrigen oxidativen Zusammensetzung B, um eine gebrauchsfertige Färbezusammensetzung mit einem pH-Wert im Bereich von 7 bis 12 zu bilden,
ix) Auftragen der gebrauchsfertigen Färbezusammensetzung auf Keratinfasern und Einwirkenlassen für eine Zeitspanne von 1 min bis 60 min,
x) Wiederholung von Schritt vi).

## Revendications

1. Composition décolorante A pour fibres kératiniques, de préférence pour fibres kératiniques humaines, plus préférentiellement pour cheveux humains, comprenant
a) un ou plusieurs persalt(s) et/ou sel(s) de peroxy,
b) un ou plusieurs agents alcalinisants,
c) un ou plusieurs alcools de sucre non acétylés et/ou leurs mélanges,
dans lequel la concentration totale de composé(s) selon le groupe c) est comprise entre 0.0001% et 2.5% en poids, calculée par rapport au poids total de la composition de décoloration A,
dans laquelle la composition décolorante A comprend moins de 5% en poids d'eau, par rapport au poids total de la composition décolorante A,
et dans lequel un ou plusieurs composé(s) selon le groupe c) est/sont du mannitol, du xylitol, et/ou leurs mélanges.

2. La composition selon la revendication 1, **caractérisée par le fait qu'**elle comprend le(s) composé(s) selon le groupe a) à une concentration totale comprise entre 10% et 80% en poids, de préférence entre 15% et 70% en poids, plus préférentiellement entre 20% et 60% en poids, encore plus préférentiellement entre 25% et 60% en poids, calculée par rapport au poids total de la composition A.

3. Composition selon les revendications 1 et/ou 2, **caractérisée par le fait que** les composés du groupe b) sont des agents alcalinisants inorganiques, de préférence choisis parmi les métasilicates, les carbonates et/ou les bicarbonates, et/ou leurs sels alcalins ou alcalins terrestres, et/ou leurs mélanges, plus préférentiellement le composé du groupe b) est le métasilicate de sodium.

4. Composition selon l'une quelconque des revendications précédentes, **caractérisée par le fait que** les composés du groupe b) sont des agents alcalinisants organiques, de préférence choisis parmi les alkylamines ou les alcanolamines selon la structure générale suivante 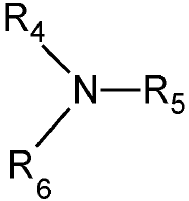 où R₄, R₅, et R₆ sont identiques ou différents de H, de C₁ à C₄, C₃ à C₄ alkyle insaturé, C₃ à C₄ alkyle ramifié, C₁ à C₄ alkyle hydroxyle, C₃ à C₄ alkyle hydroxyle insaturé, C₃ à C₄ alkyle hydroxyle ramifié, à condition qu'au moins un des R₄, R₅, ou R₆ soit différent de H, et/ou leurs mélanges, plus préférentiellement les composés selon b) sont choisis parmi la monoéthanolamine et/ou le 2-aminométhylpropanol.

5. La composition selon l'une quelconque des revendications précédentes, **caractérisée par le fait que** la composition comprend un ou plusieurs composés du groupe b) à une concentration totale comprise entre 0.25% et 30% en poids, de préférence entre 0.5% et 25% en poids, plus préférentiellement entre 1% et 20% en poids, calculée par rapport au poids total de la composition A.

6. Composition selon l'une quelconque des revendications précédentes, **caractérisée par le fait que** le ou les alcools de sucre non acétylés en tant que composés selon le groupe c) est le mannitol.

7. La composition selon l'une des revendications précédentes est **caractérisée par le fait que** la concentration totale de composé(s) selon le groupe c) est comprise entre 0.0005% et 0.5% en poids, de préférence entre 0.001% et 0.25% en poids, plus préférentiellement entre 0.001% et 0.1 % en poids, calculée par rapport au poids total de la composition A.

8. La composition selon l'une des revendications précédentes **caractérisée par le fait qu'**il s'agit d'une composition de poudre décolorante ou une composition de pâte décolorante.

9. La composition selon l'une des revendications précédentes **caractérisée par le fait qu'**elle comprend un ou plusieurs composé(s) lipophile(s) en tant que composé selon le groupe d).

10. Composition selon la revendication 9 **caractérisée par le fait que** le composé selon le groupe d) est choisi parmi les alcools gras en C₁₂ à C₂₂, les esters des alcools en C₃ à C₁₂ avec les acides gras en C₁₂ à C₂₂, les acides gras en Ca à C₂₂, les huiles végétales, et/ou les silicones, et/ou les produits à base d'hydrocarbures, et/ou leurs mélanges.

11. La composition selon l'une des revendications 9 à 10 **caractérisée par le fait que** la concentration des composés selon le groupe d) est comprise entre 1% et 20% en poids, de préférence entre 2% et 15% en poids, plus préférentiellement entre 3% et 12% en poids, calculée par rapport au poids total de la composition A.

12. Composition décolorante en deux parties pour fibres kératiniques, de préférence pour fibres kératiniques humaines, plus préférentiellement pour cheveux humains, comprenant une composition décolorante A selon l'une quelconque des revendications 1 à 11 et une seconde composition oxydante aqueuse B, comprenant de préférence du peroxyde d'hydrogène et ayant un pH compris entre 1 et 6.

13. Kit de pièces pour la décoloration et la teinture des fibres kératiniques, de préférence des fibres kératiniques humaines, et plus particulièrement des cheveux humains, comprenant :
- une composition décolorante A telle que définie dans l'une des revendications 1 à 11,
- une deuxième composition oxydante aqueuse B, comprenant de préférence du peroxyde d'hydrogène et ayant un pH compris entre 1 et 6,
- une troisième composition comprenant un ou plusieurs colorants, de préférence un ou plusieurs colorants capillaires oxydants et/ou un ou plusieurs colorants capillaires directs, et/ou leurs mélanges.

14. Méthode de décoloration des fibres kératiniques, de préférence des fibres kératiniques humaines, et plus particulièrement des cheveux humains, comprenant les étapes suivantes :
i) mélanger une composition décolorante A telle que définie dans l'une quelconque des revendications 1 à 11 avec une seconde composition oxydante B, comprenant de préférence du peroxyde d'hydrogène et ayant un pH compris entre 1 et 6, pour obtenir une composition prête à l'emploi ayant un pH compris entre 7 et 12,
ii) appliquer la composition prête à l'emploi sur les fibres kératiniques et la laisser agir pendant une période de 1 à 60 minutes,
iii) rincer les fibres kératiniques avec de l'eau et, éventuellement, les shampouiner.

15. Méthode de teinture des fibres kératiniques, de préférence des fibres kératiniques humaines, et plus particulièrement des cheveux humains, comprenant les étapes suivantes :
iv) fournir la composition décolorante A telle que définie dans l'une quelconque des revendications 1 à 11 et la mélanger avec une composition oxydante aqueuse B, comprenant de préférence du peroxyde d'hydrogène et ayant un pH compris entre 1 et 6, pour obtenir une composition prête à l'emploi ayant un pH compris entre 7 et 12,
v) appliquer la composition prête à l'emploi sur les fibres kératiniques et la laisser agir pendant une période de 1 à 60 minutes,
vi) rincer les fibres kératiniques avec de l'eau et, éventuellement, les shampouiner,
vii) fournir une composition tinctoriale comprenant un ou plusieurs colorants oxydants et/ou un ou plusieurs colorants directs, et/ou leurs mélanges, et comprenant un ou plusieurs agents alcalinisants,
viii) mélanger la composition de l'étape vii) avec une composition oxydante aqueuse B pour former une composition tinctoriale prête à l'emploi dont le pH est compris entre 7 et 12,
ix) appliquer la composition tinctoriale prête à l'emploi sur les fibres kératiniques et la laisser agir pendant une durée comprise entre 1 et 60 minutes,
x) répéter l'étape vi).
